# EUROPEAN PATENT SPECIFICATION

(11) **EP 1 501 581 B1**
(45) Date of publication and mention of the grant of the patent: **22.07.2009**
(21) Application number: 03726447.0
(22) Date of filing: 25.04.2003
(51) Int. Cl.: A61M 29/00

(54) **MEDICAL BALLOON SYSTEM FOR TREATING OBESITY**
MEDIZINISCHES BALLONSYSTEM ZUR BEHANDLUNG VON ADIPOSITAS
SYSTEME DE BALLON MEDICAL POUR TRAITEMENT DE L'OBESITE PATHOLOGIQUE

(30) Priority: 09.05.2002 US 379540 P
(43) Date of publication of application: 02.02.2005
(73) Proprietor: Reshape Medical, Rancho Santa Margarita, CA 92688 (US)
(72) Inventor: Alverdy, John C., Glenview, IL 60025 (US)
(74) Representative: Fitchett, Stuart Paul
(86) International application number: PCT/US2003/012782
(87) International publication number: WO 2003/095015

(56) References cited:
- US-A- 5 259 399
- US-A- 5 993 473

## Description

### BACKGROUND OF THE INVENTION

### Field of the Invention

This invention relates to a system for the treatment of morbid obesity and, more specifically, to a system for treating morbid obesity using a variably cycled percutaneous balloon implanted in the gastric cavity.

### Discussion of the Prior Art

Morbid obesity is a major health problem confronting the general public and health care industry today. It is estimated that approximately 50% of the U.S. population is overweight and over ten million Americans are more than 100 pounds over their ideal weight. Generally, a person is considered morbidly (or seriously) obese if they are 100 pounds or more over their ideal weight. The morbidly obese group faces increased health risks including a higher likelihood of heart disease, hypertension, diabetes and certain cancers. Over 300,000 Americans die of obesity related illnesses each year. In addition, the morbidly obese generally have lower self-esteem and are more likely to suffer from depression than the general public.

Most obese individuals have struggled unsuccessfully with their weight for a lifetime. The numerous diets, behavioral therapy and treatments such as hypnosis, pituitary hormones and appetite suppressant drugs attest to the great difficulty many overweight people have in losing weight and keeping it off. Some of these weight loss strategies can be successful in the mildly obese people, but nearly all fail in individuals considered morbidly obese. These disappointing results have led many patients and their doctors to consider surgery as an option for weight loss.

Surgical techniques bring about weight loss primarily by limiting how much the stomach can hold. Today's most common surgical procedures to promote weight loss focus on decreasing food intake by restriction. Gastric banding, gastric bypass and vertical-banded gastroplasty are surgeries that limit the amount of food the stomach can hold by closing off or removing parts of the stomach. Other surgeries attempt to permanently fill the stomach with an inflated balloon. These treatments are invasive, require major surgery with hospitalization and are associated with complications.

The success rates of current treatments and procedures have been poor. With the restrictive procedure, the patient is usually limited to eating very small amounts of food at a time. For many people, this can create a "satisfied" feeling, but they often do not feel "full". The ability to eat a large amount of food at one time is lost; consequently, many patients return to eating excessive amounts of high calorie or high sugar liquid foods. Essentially, their diet includes milk shakes and ice cream.

As to the balloon procedure of the past, very limited positive results were achieved. The balloon was relatively small when compared to the overall volume of the morbidly obese stomach. This is due to physiological limitation on the balloon volume. That is, complications of the device precluded enlarging it to a volume that would occupy more of the stomach. Yet, in order for the balloon to achieve a patient's feeling of fullness and satiation, the balloon would need to occupy a large portion (volume) of the patient's stomach. A balloon occupying this much volume without fixation or an inflation/deflation cycling has the potential of blocking food flow and causing necrosis of the stomach wall, ulcers and/or bleeding.

Moreover, success depends on the ability of a treatment to "normalize" not only the mechanical and neurohormonal sensation of feeling full and satiated, but also involves psychological factors. Both the mechanical and neurohormonal factors relate to one's need to feel "full" and "satiated". Chemicals released by the stomach during the digestive process largely drive these factors. In other words, filling the stomach or limiting its pouch size controls these chemicals. Current surgical approaches, however, fail to achieve this global feeling of "satiety" response as they restrict food entry only into the small proximal stomach pouch and bypass the distal stomach where most of the neurohormonal chemical are normally released. Medical therapy is focused almost exclusively at the brain level and is likely to continue to fail as patients experience mood disorders and complications from medications. Accordingly, there is a need for a system and method for treating morbid obesity by restoring or normalizing the appropriate "fullness signals" from the stomach itself as this is the organ that regulates fullness. In particular, the system and method of the invention should cause a feeling of satiety from the stomach itself with less consumption of food by a morbidly obese patient.

US Patent 5,259,399 and 5,993,473 disclose systems which partly addresse the above problem which use an inflatable bladder of a predetermined size and shape to substantially occupy the upper part of the stomach including the fundus.

### SUMMARY OF THE INVENTION

According to the present invention there is provided a medical system for the treatment of morbid obesity, comprising: an inflatable balloon to be implanted in a gastric cavity; and a percutaneous fillant delivery tube having a proximal end a distal end connected to the balloon, a control module connected to the proximal end of the tube for controlling the inflation and deflation of the balloon; wherein the balloon has been individually designed to meet specific morphologic features of a patient's stomach and to occupy the vast majority of the stomach, and that the control module is arranged to cyclically inflate and deflate of the balloon, and that said tube being suitable for being passed through and affixed to an abdominal wall and the gastric cavity.

A system in accordance with the present invention using a variably cycled percutaneous balloon implanted in the gastric cavity can elicit signals directly from the entire stomach in order to cause a feeling of satiety with less food. This has the potential to offer a less invasive, more complete elicitation of the feeling of fullness in patients who chronically, and perhaps genetically overeat, the system of the invention including a balloon device that is contoured to occupy the vast majority of the volume of the stomach. The system may also have the capacity to automatically inflate and deflate the balloon, thereby avoiding the problem of pressure induced injury. With the advent of CT scanning and 3-dimensional imaging, patients may have balloons individually designed to meet the specific morphologic features of their stomachs. By fixation of the balloon device, the problems of migration and obstruction are avoided. Furthermore, the system and process of the invention apply appropriate inflation/deflation cycling with a computerized device so as to avoid complications of past devices.

These and other features and advantages of the invention will become more apparent with a discussion of preferred embodiments in reference to the associated drawings.

### DESCRIPTION OF THE DRAWINGS

FIG. 1 illustrates a schematic view of a variably cycled percutaneous balloon placed within the gastric cavity of an individual in accordance with an embodiment of the invention; and
FIG. 2 illustrates a cross-sectional view of an inflatable balloon and a fillant delivery tube according to the present invention.

### DESCRIPTION OF PREFERRED EMBODIMENTS AND BEST MODE OF THE INVENTION

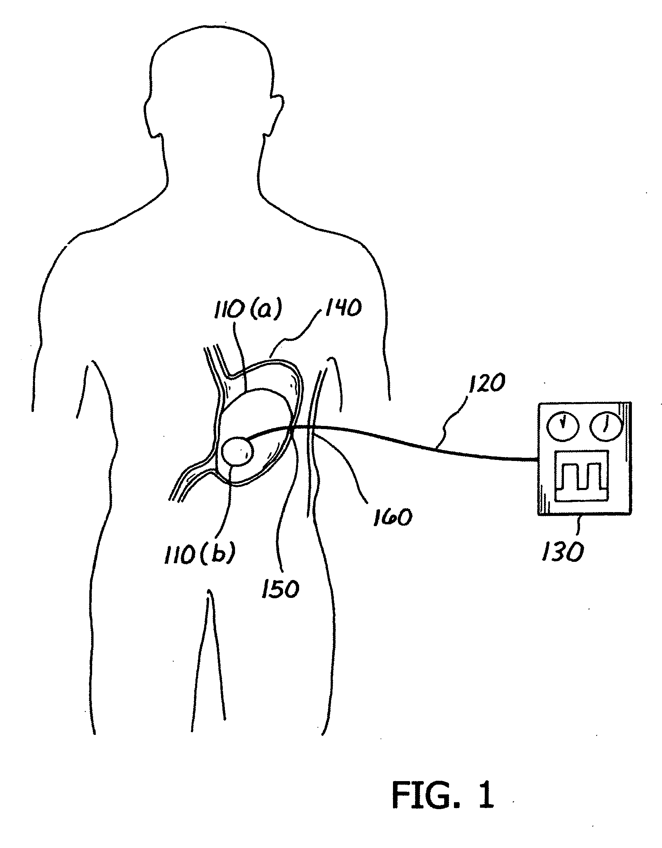

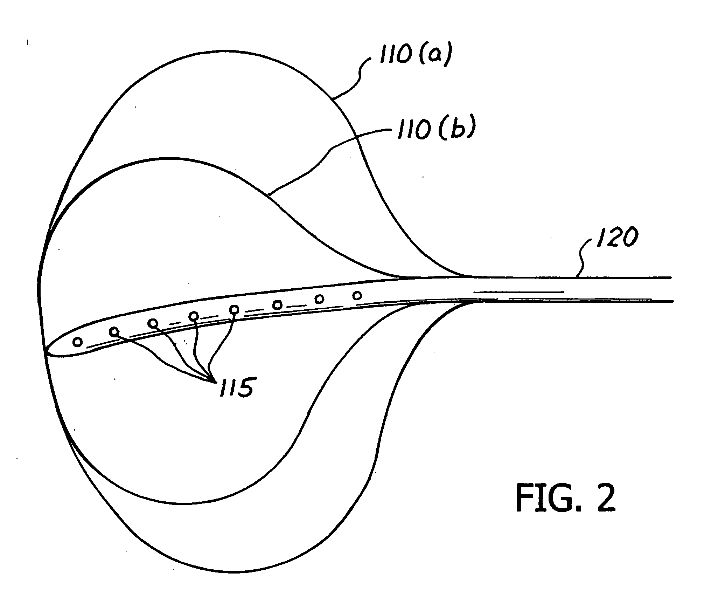

With reference to FIGS. 1 and 2, a variably cycled percutaneous balloon system for treating morbid obesity is illustrated and comprises an inflatable balloon 110 individually contoured to each patient's stomach, a percutaneous inflation or fillant delivery tube 120 having a proximal end and a distal end connected to the balloon 110, and a control module 130 connected to the proximal end of the tube 120. The tube 120 includes at least one opening 115 for filling the balloon 110 with a biocompatible fillant. The control module 130 variably controls the inflation and deflation of the balloon 110 with the biocompatible fillant such as a liquid, gas, gel or a mixture thereof. In accordance with the teachings of the present invention, the tube 120 is passed through and affixed to abdominal wall 160 and stomach wall 150. The balloon 110 is then positioned into the stomach or gastric cavity 140. The positioning of the balloon 110 may be done, e.g., by the percutaneous endoscopic gastrostomy (PEG) technique, which is known in the art. The balloon 110 and tube 120 may be separate or integral components that are constructed from any surgical grade material. For example, the balloon 110 may be made from latex rubber which expands upon introduction of a fillant, and the tube 120 may be constructed of a metal or plastic material. The tube 120 is connected to the control module 130, which may be a fixed unit or a portable unit mounted to the patient's side. The control module 130 may be a personal computer such as a desktop computer, a laptop computer or a handheld computer. The control module 130 further includes a device such as a pump for introducing and removing a fillant to and from the balloon 110.

A novel feature of the system 100 is it variably controls the inflation and deflation of the balloon 110. For example, the system 100 may inflate and deflate the balloon 110 throughout a predetermined period of time such as a 24-hour period. The balloon 110 would occupy a large volume of the stomach 140 (as shown by reference number 110(a)) when it would be most beneficial for weight loss, and deflate to give the stomach lining a rest (as shown by reference number 110(b)) during less critical time, e.g., during sleeping time. Furthermore, an algorithm tailored to each patient's needs and programmed into the control module 130 is used to control the balloon size to minimize the desire to eat and to prevent blockage or stomach lining necrosis. Unlike the restrictive procedures of the prior art, the variable inflated balloon 110 would not limit nutrient absorption and not lead to altered food choices. This is achieved as the balloon 110 contacts a major portion of the stomach wall 150 when the balloon 110 is fully inflated. Thus, the system of the invention creates a feeling of fullness and satiation by balancing the physiological, neurohormonal and chemical factors.

It will be understood that many modifications can be made to the disclosed embodiments without departing from the scope of the claims. As such, the above description should not be construed as limiting the invention, but should be interpreted as merely exemplary of preferred embodiments.

## Claims

1. A medical system (110) for the treatment of morbid obesity, comprising:
an inflatable balloon (126) to be implanted in a gastric cavity; and
a percutaneous fillant delivery tube having a proximal end a distal end connected to the balloon,
a control module connected to the proximal end of the tube for controlling the inflation and deflation of the balloon;
**characterized in that** the balloon has been individually designed to meet specific morphologic features of a patient's stomach and to occupy the vast majority of the stomach, that the control module is arranged to cyclically inflate and deflate the balloon, and that said tube being suitable for being passed through and affixed to an abdominal wall (130) and the gastric cavity.

2. The medical system of Claim 1, further comprising a biocompatible fillant of at least one of a liquid, a gas and a gel for inflating the balloon.

3. The medical system of Claim 1, wherein the control module is a fixed unit or a portable unit.

4. The medical system of Claim 1, wherein the control module is a desktop computer.

5. The medical system of Claim 1, wherein the control module is a laptop computer or a handheld computer.

6. The medical system of Claim 1, wherein the control module is programmed to inflate and deflate the balloon over a predetermined period of time.

7. The medical system of Claim 1, wherein the control module is arranged to deflate the balloon to give the gastric cavity lining a rest during less critical time.

8. The medical system of Claim 1, wherein the control module further comprises a pump for introducing and removing a fillant to and from the balloon.

9. The medical system of Claim 2, wherein the individually contoured balloon is configured to expand to a specific shape.

10. The medical system of Claim 1, wherein the balloon and the tube are integral.

## Patentansprüche

1. Medizinisches System (110) zur Behandlung von morbider Fettleibigkeit, umfassend:
Einen aufblasbaren Ballon (110a), der in einen gastrischen Hohlraum zu implantieren ist; und
einen perkutanen Füllmittelzufuhrschlauch (120) mit einem proximalen Ende, wobei das distale Ende an den Ballon angeschlossen ist,
ein Regelmodul (130), das ans proximale Ende des Schlauchs angeschlossen ist, um das Füllen und Entleeren des Ballons zu regeln;
wobei der Ballon individuell konstruiert worden ist, um spezifischen morphologischen Merkmalen des Magens eines Patienten zu entsprechen und den größten Teil des Magens zu einzunehmen und besagter Schlauch geeignet ist, durch eine Bauchwand (150) geleitet und an dieser und dem gastrischen Hohlraum befestigt zu werden,
**dadurch gekennzeichnet, dass** das Regelmodul eingerichtet ist, den Ballon zyklisch zu füllen und zu entleeren.

2. Medizinisches System nach Anspruch 1, das weiter ein biokompatibles Füllmittel zumindest jeweils eine Flüssigkeit, ein Gas und ein Gel zum Füllen des Ballons umfasst.

3. Medizinisches System nach Anspruch 1, wobei das Regelmodul eine fixierte oder eine tragbare Einheit ist.

4. Medizinisches System nach Anspruch 1, wobei das Regelmodul ein Desktopcomputer ist.

5. Medizinisches System nach Anspruch 1, wobei das Regelmodul ein Laptopcomputer oder ein handgehaltener Computer ist.

6. Medizinisches System nach Anspruch 1, wobei das Regelmodul programmiert ist, den Ballin über eine vorbestimmte Zeitspanne zu füllen und zu entleeren.

7. Medizinisches System nach Anspruch 1, wobei das Regelmodul eingerichtet ist, den Ballon zu entleeren, um der Auskleidung des gastrischen Hohlraums, während weniger kritischer Zeit Ruhe zu gönnen.

8. Medizinisches System nach Anspruch 1, wobei das Regelmodul weiter eine Pumpe zum Einführen eines Füllmittels in den und dessen Entfernen aus dem Ballon umfasst.

9. Medizinisches System nach Anspruch 2, wobei der individuell konturierte Ballon konfiguriert ist, sich zu einer spezifischen Form auszudehnen.

10. Medizinisches System nach Anspruch 1, wobei der Ballon und der Schlauch integral sind.

## Revendications

1. Un système médical (110) destiné au traitement de l'obésité pathologique, comprenant :
un ballon gonflable (110a) à implanter dans la cavité gastrique ; et un tube de libération d'agent de remplissage par voie percutanée (120) ayant une extrémité proximale et une extrémité distale connectée au ballon,
un module de commande (130) connecté à l'extrémité proximale du tube pour la commande du gonflement et du dégonflement du ballon ;
selon lequel le ballon a été conçu individuellement afin de répondre à des caractéristiques morphologiques spécifiques de l'estomac du patient et afin d'occuper la vaste majorité de l'estomac, ledit tube étant adapté pour être passé dans l'estomac et fixé à une paroi abdominale (150) et à la cavité gastrique, **caractérisé en ce que** le module de commande est agencé de manière à gonfler et à dégonfler cycliquement le ballon.

2. Système médical de la Revendication 1, comprenant en outre un agent de remplissage biocompatible composé d'au moins un d'un liquide, d'un gaz et d'un gel pour le gonflement du ballon.

3. Système médical de la Revendication 1, le module de commande étant une unité fixe ou une unité portable.

4. Système médical de la Revendication 1, le module de commande étant un ordinateur portable.

5. Système médical de la Revendication 1, le module de commande étant un ordinateur portable ou un ordinateur de poche.

6. Système médical de la Revendication 1, le module de commande étant programmé de manière à gonfler et à dégonfler le ballon durant une période de temps prédéterminée.

7. Système médical de la Revendication 1, le module de commande étant agencé de manière à dégonfler le ballon afin de conférer du repos à la muqueuse gastrique durant les périodes moins critiques.

8. Système médical de la Revendication 1, le module de commande comprenant en outre une pompe pour l'introduction et le retrait d'un agent de remplissage dans et à partir du ballon.

9. Système médical de la Revendication 2, le ballon individuellement formé étant configuré pour une expansion à une forme spécifique.

10. Système médical de la Revendication 1, le ballon et le tube faisant partie intégrante l'un à l'autre.
